# FASCICULE DE BREVET EUROPEEN

(11) **EP 0 367 677 B1**
(45) Date de publication et mention de la délivrance du brevet: **04.08.1993**
(21) Numéro de dépôt: 89403026.1
(22) Date de dépôt: 03.11.1989
(51) Int. Cl.: A61M 5/30

(54) **Dispositif d'injection sous cutanée sans aiguille**
Vorrichtung für subkutane nadellose Injektion
Device for subcutaneous needle-less injection

(30) Priorité: 03.11.1988 FR 8815376
(43) Date de publication de la demande: 09.05.1990
(73) Titulaire: Dalto, Tino, F-06000 Nice (FR); Laruelle, Claude, F-06270 Villeneuve-Loubet (FR)
(72) Inventeur: Dalto, Tino, F-06000 Nice (FR); Laruelle, Claude, F-06270 Villeneuve-Loubet (FR)
(74) Mandataire: Ramey, Daniel

(56) Documents cités:
- EP-A- 0 276 158
- FR-A- 2 620 338
- GB-A- 677 523
- GB-A- 1 333 215
- US-A- 2 681 653
- US-A- 3 140 713
- US-A- 3 788 315
- US-A- 3 802 430
- ENGINEERING, vol. 211, no. 8, novembre 1971, pages 901-905; P.N. BISSEL et al.:"Exit the needle"

## Description

L'invention concerne un dispositif d'injection sous-cutanée, sans aiguille.

On connaît, depuis longtemps, des dispositifs de ce type, qui comprennent un corps tubulaire dans lequel est guidé un piston en mouvement alternatif. Une extrémité ouverte du corps tubulaire reçoit la tête d'injection comprenant un trou de très petit diamètre, en général compris entre 0,2 et 1 mm. Le piston logé dans le corps tubulaire est associé à un mécanisme de poussée par ressort, comportant des moyens de verrouillage en position comprimée et de détente ou déverrouillage.

Le produit médicamenteux à injecter est aspiré à l'extrémité ouverte du corps tubulaire et contenu dans cette extrémité. La tête d'injection est ensuite montée sur cette extrémité du corps tubulaire, puis le déverrouillage du mécanisme de poussée chasse, au moyen du piston, le produit à travers le trou très fin de la tête d'injection qui est appliquée sur la peau du patient. Le produit médicamenteux est alors pulvérisé de façon très fine et passe à travers la peau du patient.

On connaît, par le document US-A-3 802 430 un dispositif d'injection de ce type, à commande pyrotechnique, qui comprend un piston guidé en déplacement dans un corps tubulaire se terminant par une tête d'injection comportant plusieurs trous parallèles. La mise à feu d'une charge pyrotechnique propulse le piston dans le corps tubulaire pour expulser un produit médicamenteux par les trous parallèles de la tête d'injection.

Ce mode d'injection de produits médicamenteux est moins traumatisant que l'utilisation d'une seringue classique à l'aiguille. Cependant, on constate souvent que la peau du patient est abîmée au point d'injection, et qu'il se forme rapidement des hématomes dûs à la rupture de vaisseaux sanguins. Le patient peut également ressentir une douleur assez vive au moment de l'injection du produit.

La présente invention a notamment pour but d'éviter ces inconvénients.

Elle a pour objet un dispositif d'injection sous cutanée sans aiguille, permettant de réduire dans une large mesure la douleur ressentie au moment de l'injection, de ne pas abîmer la peau du patient, et de ne pas créer d'hématome, tout en assurant cependant l'injection d'une dose prédéterminée de produit médicamenteux.

Elle propose à cet effet un dispositif d'injection sous-cutanée sans aiguille, comprenant un corps tubulaire à une extrémité duquel est montée une tête d'injection comprenant plusieurs trous d'injection parallèles et perpendiculaires à la surface de la tête d'injection sur laquelle ils débouchent, un piston guidé en déplacement dans le corps, un mécanisme de poussée du piston, logé dans le corps et associé à des moyens de verrouillage et à des moyens de détente, caractérisé en ce que chaque trou d'injection a un diamètre inférieur à 100 microns environ et est situé à l'extrémité d'un conduit cylindrique formé dans l'épaisseur de la tête d'injection, ces conduits cylindriques ayant un diamètre supérieur à celui des trous d'injection, par exemple un diamètre compris entre 0,5 et 1 mm, et étant réunis entre eux à leurs extrémités tournées vers le piston et débouchant sur une surface concave de la tête d'injection, dont la forme est conjuguée de celle de l'extrémité correspondante du corps tubulaire.

La présence de plusieurs trous d'injection très fins, ayant un diamètre inférieur à 100 microns environ, dans la tête précitée, permet de réduire la durée de l'injection du produit médicamenteux, le risque de formation d'hématomes et la douleur ressentie par le patient. Le diamètre très faible des trous d'injection permet de pulvériser très finement le produit médicamenteux et d'aider son passage à travers la peau du patient.

Par ailleurs, la disposition des trous d'injection aux extrémités de conduits cylindriques de plus grand diamètre qui sont formés dans l'épaisseur de la tête d'injection, facilite la réalisation des trous d'injection de diamètre très faible.

Selon encore une autre caractéristique de l'invention, la tête d'injection est réalisée en acier inoxydable et les trous d'injection ont une longueur au moins égale à 0,5 mm.

Cette caractéristique est importante, dans la mesure où une longueur plus faible des trous d'injection risque de se traduire par un éclatement du métal au niveau de ces trous, en raison de la très forte pression d'injection à travers ces trous.

Selon un mode de réalisation préféré de l'invention, la tête d'injection est sertie sur une bague à filetage interne, permettant son montage par vissage sur l'extrémité du corps tubulaire.

La tête d'injection est ainsi interchangeable, et peut être stérilisée facilement. En outre, on peut choisir une tête d'injection d'un type particulier, en fonction du produit à injecter, et de la localisation de la zone d'injection sur le corps du patient.

Par exemple, les trous d'injection peuvent être répartis sur un cercle ayant un diamètre de 8 à 10 mm environ, en particulier pour l'injection d'insuline, ou bien sur un cercle ayant un diamètre compris entre 2 et 4 mm environ, en particulier pour l'injection d'anesthésique en chirurgie dentaire, ou encore sur un cercle ayant un diamètre compris entre 15 et 20 mm environ, en particulier en mésothérapie.

L'invention sera mieux comprise et d'autres caractéristiques, détails et avantages de celle-ci apparaîtront plus clairement à la lecture de la description qui suit, faite à titre d'exemple en référence aux dessins annexés dans lesquels :
- la figure 1 représente schématiquement un dispositif selon l'invention;
- la figure 2 est une vue partielle agrandie, en coupe longitudinale, de l'extrémité du corps tubulaire du dispositif de la figure 1;
- la figure 3 est une vue, encore agrandie, en coupe longitudinale d'une tête d'injection se montant par vissage sur l'extrémité du corps tubulaire représenté en figure 2.

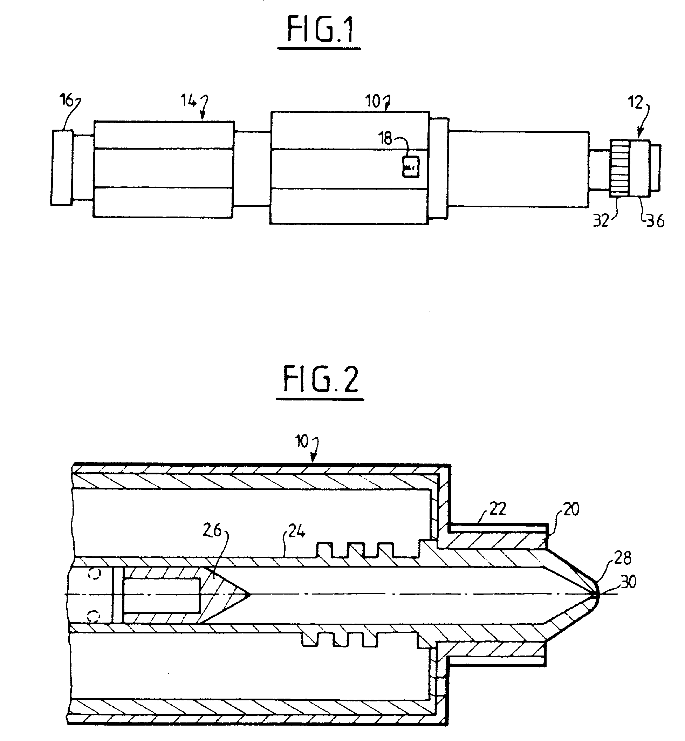

On fera tout d'abord référence à la figure 1, qui est une vue schématique d'ensemble d'un dispositif d'injection selon l'invention.

Ce dispositif comprend un corps tubulaire désigné par la référence 10 et à une extrémité duquel est montée une tête d'injection 12 selon l'invention. Un mécanisme de poussée à ressort, d'un type classique, est logé dans un cylindre 14 et est associé à des moyens de verrouillage en compression du ressort et à des moyens de déverrouillage ou de détente, par exemple du type à bouton poussoir 16. Ce mécanisme de poussée agit sur un piston guidé par déplacement dans le corps tubulaire 10 et destiné à propulser un produit médicamenteux à travers des trous extrêmement fins de la tête d'injection 12.

De façon connue, le mécanisme de poussée 14 est, dans son ensemble, monté vissable à l'intérieur du corps 10, sa position longitudinale à l'intérieur de ce dernier et déterminant la dose de produit à injecter. Par exemple, le corps tubulaire 10 peut comprendre une petite fenêtre 18 à travers laquelle apparaissent des graduations portées par une partie du mécanisme 14 et correspondant à des quantités de produit à injecter.

On se réfère maintenant à la figure 2 dans laquelle on voit l'extrémité du corps 10 sur laquelle la tête d'injection 12 est montée par vissage.

L'extrémité du corps 10 comprend à cet effet un prolongement cylindrique tubulaire 20 à filetage extérieur 22, qui reçoit à étanchéité l'extrémité d'un tube cylindrique 24 dans lequel est guidé le piston 26 de propulsion du produit à injecter. L'extrémité libre du tube 24 est de forme sensiblement conique dont le sommet 28 arrondi comporte un trou axial 30 de faible diamètre, par exemple de l'ordre de 1 mm.

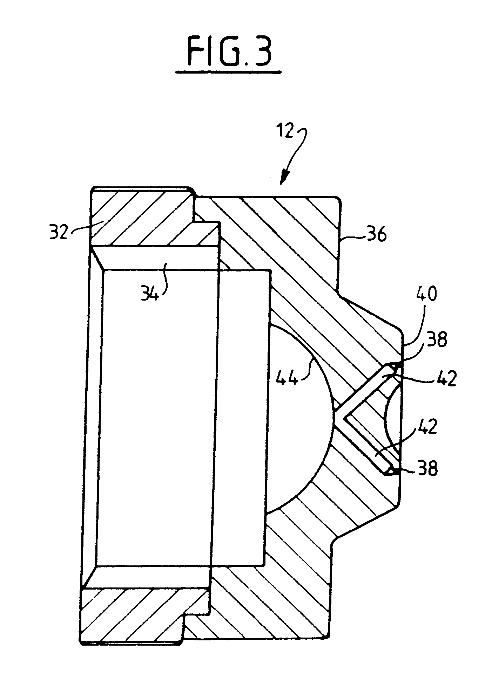

La tête d'injection 12, représentée à plus grande échelle et en coupe longitudinale en figure 3, comporte une bague annulaire 32 à filetage interne 34 permettant son montage par vissage sur l'extrémité cylindrique 20 du corps 10, et une tête d'injection proprement dite 36, en forme de capeau, qui est fixée, par exemple par sertissage, sur la bague annulaire 32.

Cette pièce 36 réalisée en acier inoxydable, comprend plusieurs trous d'injection 38 de diamètre très faible, inférieur à 100 microns, et compris par exemple entre 10 et 100 microns.

Ces trous 38 sont parallèles entre eux et orientés perpendiculairement à la face extérieure plane 40 de la tête d'injection (la face 40 constituant la face d'application de la tête d'injection sur la peau d'un patient).

Pour des raisons de commodité de fabrication, les trous d'injection 38 sont rectilignes et formés aux extrémités de conduits cylindriques 42 de plus grand diamètre (par exemple compris entre 0,5 et 1 mm) qui sont eux orientés en oblique et qui sont réunis entre eux à leur extrémité interne, sensiblement sur l'axe longitudinal de la tête 12.

Les conduits cylindriques 42 débouchent par leur extrémité interne commune sur une surface concave arrondie 44 de la pièce 36, cette surface 44 ayant un rayon de courbure correspondant sensiblement à celui de l'extrémité arrondie 28 du tube 24 représenté en figure 2. De la sorte, lorsque la bague 12 est montée par vissage sur l'extrémité filetée 20 du corps 10, le nez arrondi 28 du tube 24 vient s'appliquer sur la surface concave arrondie 44 de la tête 12, le trou axial 30 du tube 24 se trouvant directement aligné avec l'extrémité commune des conduits cylindriques 32.

Il est par ailleurs important que les trous d'injection 38 aient une longueur d'au moins 0,5 mm, pour éviter l'éclatement du métal de la pièce 36 lors de l'injection du produit.

Les trous d'injection 38 sont percés dans la tête d'injection au moyen de forets très fins. D'autres techniques peuvent être utilisées, par exemple l'électro-érosion ou un faisceau laser.

Le nombre de trous d'injection 38 peut varier entre 2 et 10 environ, ces trous étant répartis sur un cercle dont le rayon peut varier assez largement en fonction du type de produit à injecter, et aussi de la localisation du point d'injection sur le corps des patients.

Par exemple, dans le cas de l'injection d'insuline, le nombre de trous 38 peut être de 2 ou 3, ces trous étant répartis sur un cercle ayant un diamètre compris entre 8 et 10 mm environ.

Pour l'injection d'un anesthésique en chirurgie dentaire, les trous seront répartis sur un cercle plus petit, ayant un diamètre compris entre 2 et 4 mm environ.

En mésothérapie, le nombre de trous sera plus important et les trous seront répartis sur un cercle ayant un diamètre compris entre 15 et 20 mm environ.

On a constaté que la tête d'injection selon l'invention permettait d'injecter des produits médicamenteux de façon pratiquement indolore, sans lésion de la peau et sans apparition d'hématome. Lorsque la pression d'injection est correctement réglée, ce que l'on obtient par vissage ou dévissage du mécanisme de poussée 14, la peau reste sèche après l'injection, ce qui signifie que le produit a été entièrement injecté sous la peau et n'a pas tendance à ressortir vers l'extérieur. En outre, on ne constate en général pas la présence d'une gouttelette de sang au point d'injection.

## Revendications

1. Dispositif d'injection sous-cutanée sans aiguille, comprenant un corps tubulaire (10) à une extrémité duquel est montée une tête d'injection (12) comprenant plusieurs trous d'injection (38) parallèles et perpendiculaires à la surface (40) de la tête d'injection sur laquelle ils débouchent, un piston (26) guidé en déplacement dans le corps (10), un mécanisme (14) de poussée du piston, logé dans le corps (10) et associé à des moyens de verrouillage et à des moyens de détente, caractérisé en ce que chaque trou d'injection (38) a un diamètre inférieur à 100 microns environ et est situé à l'extrémité d'un conduit cylindrique (42) formé dans l'épaisseur de la tête d'injection, ces conduits cylindriques (42) ayant un diamètre supérieur à celui des trous d'injection (38), par exemple un diamètre compris entre 0,5 et 1 mm, et étant réunis entre eux à leurs extrémités tournées vers le piston et débouchant sur une surface concave (44) de la tête d'injection dont la forme est conjuguée de celle de l'extrémité correspondante (28) du corps tubulaire.

2. Dispositif selon la revendication 1 précédente, caractérisé en ce que le diamètre des trous d'injection (38) est compris entre 10 et 100 microns environ.

3. Dispositif selon la revendication 1 ou 2, caractérisé en ce que les trous d'injection (38) sont répartis sur un cercle ayant un diamètre de 8 à 10 mm environ, en particulier pour l'injection d'insuline.

4. Dispositif selon la revendication 1 ou 2, caractérisé en ce que les trous d'injection (38) sont répartis sur un cercle ayant un diamètre de 2 à 4 mm environ, en particulier pour l'injection d'anesthésique en chirurgie dentaire.

5. Dispositif selon la revendication 1 ou 2, caractérisé en ce que les trous d'injection (38) sont répartis sur un cercle ayant un diamètre de 15 à 20 mm environ, en particulier pour utilisation en mésothérapie.

6. Dispositif selon l'une des revendications précédentes, caractérisé en ce que la tête d'injection est réalisée en acier inoxydable et les trous d'injection (38) ont une longueur au moins égale à 0,5 mm.

7. Dispositif selon la revendication 6, caractérisé en ce que la tête d'injection (36) est sertie sur une bague (32) à filetage interne (34) permettant son montage par vissage sur l'extrémité filetée (20, 22) du corps tubulaire.

## Claims

1. A device for subcutaneous injection without a needle, the device comprising a tubular body (10) having an injection head (12) mounted at one end thereof, the head including a plurality of parallel injection holes (38) which are perpendicular to the surface (40) of the injection head through which they open out, a piston (26) guided to move relative to the body (10) a piston thrust mechanism (14) housed inside the body (10) and associated with locking means and with release means, the device being characterized in that each injection hole (38) has a diameter of less than about 100 microns and is situated at the end of a cylindrical duct (42) formed through the thickness of the injection head, these cylindrical ducts (42) having a diameter greater than the diameter of the injection holes (38), e.g. a diameter in the range 0.5 mm to 1 mm, and being interconnected at their ends directed towards the piston and opening out on a concave surface (44) of the injection head which is complementary in shape to the corresponding end (28) of the tubular body.

2. A device according to claim 1, characterized in that the diameter of the injection holes (38) lies in the range about 10 microns to about 100 microns.

3. A device according to claim 1 or 2, characterized in that the injection holes (38) are distributed around a circle having a diameter of about 8 mm to about 10 mm, in particular for injecting insulin.

4. A device according to claim 1 or 2, characterized in that the injection holes (38) are distributed around a circle having a diameter of about 2 mm to about 4 mm, in particular for injecting anaesthetic in dental surgery.

5. A device according to claim 1 or 2, characterized in that the injection holes (38) are distributed around a circle having a diameter of about 15 mm to about 20 mm, in particular for use in mesotherapy.

6. A device according to any preceding claim, characterized in that the injection head is made of stainless steel and the injection holes (38) are not less than 0.5 mm long.

7. A device according to claim 6, characterized in that the injection head (36) is crimped on a ring (32) having an inside thread (34) enabling it to be screwed onto the threaded end (20, 22) of the tubular body.

## Patentansprüche

1. Vorrichtung für nadellose subkutane Injektion, die aufweist: einen röhrenförmigen Körper (10), bei welchem an einem Ende ein Injektionskopf (12) angebracht ist, der aufweist: mehrere parallele Injektionsbohrungen (38), welche normal in die Oberfläche des Injektionskopfes einmünden, einen Kolben (26), der in dem Körper (10) verschieblich geführt ist, und einen Mechanismus (14) zum Verschieben des Kolbens, der in dem Körper (10) untergebracht und Verriegelungs- und Entriegelungsmitteln zugeordnet ist, **dadurch gekennzeichnet,daß** jede Injektionsbohrung (38) mit einem Durchmesser von geringer als etwa 100 µm an dem Ende einer im Inneren des Injektionskopfes ausgebildeten zylindrischen Leitung (42) liegt, wobei diese zylindrischen Leitungen (42) einen Durchmesser, beispielsweise zwischen 0,5 und 1 mm, besitzen, der größer ist als jener der Injektionsbohrungen (38), und an ihren in Richtung des Kolbens liegenden Enden untereinander verbunden sind und aus einer konkaven Oberfläche (44) des Injektionskopfes entspringen, deren Form dem entsprechenden Ende (28) des röhrenförmigen Körpers angepaßt ist.

2. Vorrichtung nach Anspruch 1, **dadurch gekennzeichnet, daß** der Durchmesser der Injektionsbohrungen (38) etwa zwischen 10 und 100 µm liegt.

3. Vorrichtung nach Anspruch 1 oder 2, **dadurch gekennzeichnet, daß** die Injektionsbohrungen (38), insbesondere zur Injektion von Insulin, auf einen Kreis mit einem Durchmesser von ungefähr 8 bis 10 mm aufgeteilt sind.

4. Vorrichtung nach Anspruch 1 oder 2, **dadurch gekennzeichnet, daß** die Injektionsbohrungen (38), insbesondere für Anästhesie-Injektionen in der Zahnchirurgie, auf einen Kreis mit einem Durchmesser von ungefähr 2 bis 4 mm aufgeteilt sind.

5. Vorrichtung nach Anspruch 1 oder 2, **dadurch gekennzeichnet, daß** die Injektionsbohrungen (38), insbesondere zur meso-therapeutischen Verwendung, auf einen Kreis mit einem Durchmesser von ungefähr 15 bis 20 mm aufgeteilt sind.

6. Vorrichtung nach einem der vorigen Ansprüche, **dadurch gekennzeichnet, daß** der Injektionskopf aus Edelstahl besteht und die Injektionsbohrungen (38) zumindest eine Länge von 0,5 mm besitzen.

7. Verfahren nach Anspruch 6, **dadurch gekennzeichnet, daß** der Injektionskopf (36) von einem Ring (32) mit einem Innengewinde (34) umgeben ist, das an dem mit einem Gewinde versehenen Ende (20, 22) des röhrenförmigen Körpers aufschraubbar ist.
